# EUROPEAN PATENT APPLICATION

(11) **EP 0 909 532 A1**
(43) Date of publication of application: **21.04.1999**
(21) Application number: 97308194.6
(22) Date of filing: 16.10.1997
(51) Int. Cl.: A01N 63/00, A01N 25/08

(54) **Environmentally compatible porous material comprising beneficial nematodes and its preparation**

(71) Applicant: DEVELOPMENT CENTER FOR BIOTECHNOLOGY, Taipei (TW)
(72) Inventor: Chu, Liang-Kuang, Taipei, Taiwan (CN); Lin, Chiao Po, Taipei, Taiwan (CN); Shih, Chien-Ming, Taipei, Taiwan (CN); Lee, Tsae Yueh, Taipei, Taiwan (CN); Soong, Tai-Sen, Taipei, Taiwan (CN)
(74) Representative: Woods, Geoffrey Corlett

(57) **Abstract**

A biotic preparation for controlling insects made of an environmentally compatible porous material, beneficial nematodes and symbiotic bacteria.

A method for controlling pests in grass, in a field or on a lawn using the biotic preparation.

## Description

The invention relates to a method for preparing an environmentally compatible porous material comprising beneficial nematodes with pesticidal activity, and to the biotic preparations produced therefrom and to the use for controlling pests.

Nematode-based pesticide is not a nematicide but a kind of novel pesticide, and a biotic preparation which comprises beneficial nematodes associates with the symbiotic bacteria thereof for controlling pests.

Nematode-based pesticides differ from chemical pesticides. For instance, general chemical pesticides are highly toxic, pesticide poisonous, and easy to result in environmental pollution; they affect natural predators, destroy ecological balance, and often cause the resistance against the targeted pests.

The beneficial nematodes which are often used in nematode-based pesticides include Rhabditida, in particular, Rhabditidae, Steinernematidae and Heterorhabditidae. Similar to most of nematodes, they have a simple life cycle comprising egg, 4 larvae stages and adult. The infective stage is "stage 3 infective juvenile (J3)", or called " infective juvenile (IJ)". IJs are especially resistant to environmental conditions, and enter into hosts through the original opening on the body of hosts, such as mouth, anus or respiration pores. The insecticidal course is described as follow: the infective nematode will actively penetrate the gut wall or trachea and enter the hemocoel, whereupon the symbiotic bacteria therein, such as *Xenorhabdus*, will be released to the host. The symbiotic bacteria will rapidly in the host body. The host will die from septicemia in about 48 hours. After uptaking bacterium cells and tissues of the host, the unmature nematode will develop to a adult (1).

The beneficial nematodes are harmless to plants and safe to human beings and domesticated animals. In 1987, the Environmental Protection Administration of United States had established that biotic preparations comprising Steinernema and Heterorhabditis and symbiotic bacteria thereof may be not necessary for the statutory requirement of FIFRA Section 25(b)(1). They are commercially available and have not be registered in many countries. They have no effects on the other non-targeted arthropods in soil, and there are no evidences for showing the development of resistance by pests.

US Patent 4,334,498 (1982) (2) disclosed a method for rearing nematodes. However, the patent provides the method for cultivating nematodes cultivate and the steps of harvesting the nematodes; but no treatment, storage and application after recovering the nematodes. Therefore, the method cannot be applied to the field to kill pests.

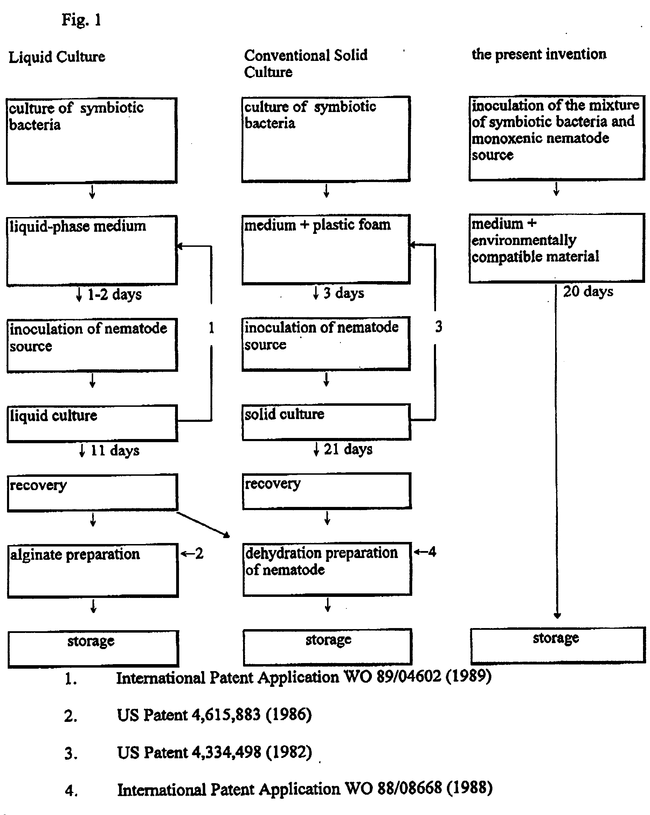

The techniques for rearing nematodes so far in the world are mostly based upon the solid and liquid culture. The most important technique of nematode-based pesticide still focuses on the technology of the mass production and the formulation of preparations. Because nematodes are multicellular animals, the preparations are hard to attain the purpose of prolonged storage as other microbiotic preparations (such as storage for one year or above). For instance, the methods described in WO 89/04602 (3) and US Patent 4,615,883 (1986) (4) (see, Fig. 1), Biosys Corp. in USA utilize alginate to embed nematodes, and can store the preparations for 3 to 6 months. However, it has to dissolve nematodes by using sodium citrate (taking about 30 minutes), and then be diluted with water before spraying onto crops. Accordingly, they are not convenient for substantial application.

The methods described in US Patent 4,334,498 and WO 88/08668 (5) (see, Fig. 1), used the principle of nematode cryptobiosis, to carry out the dehydration of nematodes under the conditions for decreasing relative humidity stepwise and to make the nematodes into clays. The storage for the clays obtained may be 3 to 6 months under refrigerated conditions. However, it spends too much time and energy in the dehydration procedure, so that the cost required will be relatively high. In addition, the clays easily harm to the lungs of human and have to be treated at high humidity (95% relative humidity) overnight for rehydrating nematodes in the next day. Therefore, the methods are too complicated to be utilized conveniently.

The methods for producing nematode-based insecticide are similar to that for fermenting general microorganisms, except the additional more complicated procedures of cultivating sterile source and the mixed cultivation along with their biosymbiotic bacteria. Because nematodes are multi-cellular animals, the cultivation periods are longer than those of cultivating other microorganisms (for example, it takes 11 and 21 days for the liquid and solid culture, respectively). As illustrated in Fig. 1, the methods used in conventional process need additional steps from recovery to storage. In particular, the recovery step is complicated and labor cost, and cause the loss in nematodes. Moreover, the recovery step always cause the problem of waste water. It presents the same situation in producing the preparation. Besides, the cultivation period of nematode is very long, and the recovery procedure takes lots of work so that the cost for production is relatively high accordingly. Therefore, the costs of nematode-based pesticidal biotic preparations tend to be high, and the price of products are also expensive. Furthermore, the methods for producing nematode-based insecticide used in conventional culture require two separable steps of cultivating symbiotic bacteria and inoculating nematodes in medium before recovery procedure. It also takes lots of work to accomplish the steps, especially for solid culture, in comparison with one-step method for cultivating symbiotic bacteria and inoculating nematodes in medium.

T.N. Wang *et al* in Sugar Research institute (1992) (6) had reported that they utilize sugar dregs to absorb nematode liquid and apply them to the field. (The nematodes may be stored at room temperature for about one month.) However, the use of bagasse as the matrix for culturing nematodes (adding culture medium) is not disclosed in the article. In addition, the bagasse will easily attacked by fungi due to sugar residue.

Georgis. R. p178, Formulation and application technology in "Entomopathogenic Nematodes in Biological Control" (7) only describes that vermiculite and peat can be used as moistened carriers for transporting and storing nematodes. However, the nematodes should be cultured and recovered prior to applying vermiculite and peat. As previously reported, the recovery step is cost-consuming, and cause the loss in nematodes and the problem of waste water. In addition, Georgis did not use vermiculite or peat as a support medium for culturing nematodes.

The disadvantages of the prior art can be minimized by the present invention.

It is an object of the present invention to provide a biotic preparation for controlling insect comprising an environmentally compatible porous material containing beneficial nematodes with pesticidal activity in which the nematodes are obtained by incubating symbiotic bacteria and the nematode source in the porous material containing production medium.

It is an other object of the present invention to provide a method for preparing an environmentally compatible porous material containing entomogenous beneficial with pesticidal activity comprising inoculating symbiotic bacteria and the nematode source in the porous material containing production medium.

It is a further object of the present invention to provide a method for controlling pests comprising treating the desired objectives to be kept free from the pests with an environmentally compatible porous material containing beneficial nematodes with pesticidal activity.

The environmentally compatible porous material may be directly applied to the desired place to eliminate the recovery procedures and formulation steps in the prior art.

The invention is further illustrated by the accompanying drawings in which

Fig. 1 describes the schemes of the methods used by conventional liquid culture and solid culture and the invention for preparing pesticidal preparations.

The following terms are used in illustrating the description, examples and claims of the invention.

The term "beneficial nematodes" refers to nematodes including Rhabditida, in particular, Rhabditidae, Steinernematidae and Heterorhabditidae, which kill hosts by its association with the symbiotic bacteria.

The term "environmentally compatible" refers to "without causing environmental contamination or poison, and agreeable to the relative regulations of environmental conservation."

The term "biodecompositable" refers to "decompositable or destructible by microorganisms or organisms occuring in the nature."

The term "porous material(s)" refers to material(s) with many pores. The porous materials used herein include environmentally compatible porous material (for example, vermiculite) or biodecompositable porous material (for example, sponge, loofah, etc.),. both of which may be useful in mass production of entomogenous nematodes and make no pollution to the public.

"controlling pests" includes repulsion and destruction. Repulsion refers to the procedure that insects or slugs will escape far away from the desired place without causing any destructive effect. Destruction refers to the procedure that nematodes will kill the insect hosts or slugs thereof by its association with the symbiotic bacteria.

For more detailed description of the technology and characteristic of the present invention, it is provided by following examples to illustrate the invention.

### Materials and Methods

Tested nematodes: *Steinernema carpocapsae* (SC) strain A11 (SCA). [After the *in vivo* culture in silkworm or tobacco cutworm, it was isolated and stored at 10°C.] The monoxenic nematodes were obtained by the method disclosed in Edward J. and Irene Popiel in Journal of Nematology 21(4):500-504, 1989(8).

Tested bacteria: the symbiotic bacteria isolated from the intestine tract of SCA, and which was identified by Food Science & Technology Research Institute in Taiwan as *Xenorhabdus nematophilus*. It was stored at -70°C.

The composition of TSA (Tryptic Soy Agar) is listed as follows:

| | |
|---|---|
| TSA (Difco) | 40 g |
| distilled water | 1 L |

The composition of YS is listed as follows:

| | |
|---|---|
| K₂HPO₄ (Merck) | 0.5 g |
| NH4·H₂PO₄ (Sigma) | 0.5 g |
| MgSO₄·7H₂O (Merck) | 0.2 g |
| NaCl (Merck) | 5.0 g |
| yeast extract (Merck) | 5.0 g |
| distilled water | 1 L |

The composition of production medium (PM) is listed as follows:

| | |
|---|---|
| egg (ordinary supermarket) | 500 g |
| yeast powder (Taiwan Sugar Corp.) | 12 g |
| Salad oil (Taiwan Sugar Corp.) | 60 g |
| distilled water | 1 L |

The sequential procedure of cultivating the symbiotic bacteria was described as follows: incubating the bacteria at -70°C in TSA; at 28°C for 24 hours; in YS liquid medium for 16 hours (at 25°C, 150 rpm, in 30 ml/125 ml flask).

The culture is thoroughly mixed with monoxenic nematode source to form a uniform mixture. Then, the mixture is inoculated into environmentally compatible porous material including production medium autoclaved at 121°C for 30 min. in a sealable vessels, for a period sufficient to the nematodes populated in the porous material (such as 15-21 days) at 25 °C to produce environmentally compatible porous material comprising the nematodes populated therein.

The environmentally compatible porous material comprising the nematodes can be directly applied to the desired place such as field, grass, etc.) for controlling pests.
Insect for Bioassay: 4th instar Tobacco cutworm (*Spodoptera litura*).
Method for Bioassay: Petri-disk and filter paper method disclosed in Jennifer L. Woodring et al, 1988 (1). The ratio of nematodes to insects = 25:1.
The plastic sponge (2 x 2 x 2 cm³) used in the tests was made of polyether polyurethane, which might be autoclaved and purchased from Jih-Hung Sponge Inc.
The vermiculite (No.2) and pearlite (No.2) used in the tests were all purchased from Nan-Hei Vermiculite Corp.

The glass containers used in the coincubation steps of nematodes and symbiotic bacteria were equipped by a 125 ml flask(Pyrex) with sponge or 2 to 5g porous material described above.

### Example 1

### The solid culture of SCA in different materials

In the example vermiculite, pearlite and foam sponge (or called sponge) were used, and the addition ratio is listed in Table 1. 10% symbiotic bacteria (i.e. 2ml) and 10⁴ monoxenic SCA were inoculated. After 14 day (25°C), the yields and the insecticidal activity were determined, and the results are listed in Table 1.

**Table 1**

| The effects on solid culture of SCA in different material | | | | |
|---|---|---|---|---|
| material | weight (g) | medium (ml) | yield (10⁴/ml) | insecticidal activity |
| vermiculite | 4.0 | 20 | 44 | 95 |
| pearlite | 5.0 | 20 | 11 | 70 |
| sponge | 2.0 | 20 | 49 | 80 |

### Example 2

### The cultivation of beneficial nematodes in environmentally compatible porous material (such as vermiculite)

A 14L vessel charged with 600 g of vermiculite and 3000 ml of PM was autoclaved for 30 minutes, then were inoculated 300 ml of symbiotic bacteria and 2 million monoxenic SCA. After being cultured for 20 days at 25 °C, the yield and pesticidal activity were determined. The results are listed in Table 2.

**Table 2**

| vessel volume | yield (10⁴/g vermiculate) | insecticidal activity |
|---|---|---|
| 14 L | 29.5 | 100 |
| flask | 30.0 | 95 |

### Example 3

### Evaluations of the biological control on larvae of tobacco cutworm by SCA presented in vermiculite preparation in the pot test

Period of test: May 24, 1994 to June 10, 1994
Testing place: the 6th balcony of Developmental Center of Biotechnology (DCB, address: 81 Chang Hsing Street, Taipei, Taiwan).
Tested nematodes: SCA₁ which were cultured in vermiculate and PM medium.
Insects to be tested: the fourth instar of *Spodoptera litura* Fabricius (tobacco cutworm) which was provided by Professor Shih Cheng-jen in Department of Botanical Blight of National Taiwan University.
Grass to be tested: *Cynodon spp*. D.W., strain 419 and strain 328, obtained from the construction site of Green in Lung Tan and Yang-Sneng golf course (Yang Mei) respectively.
Pot: 20.5 x 13.5 x 7.5 cm³, area 276.5 cm³.
Design of test: completely random design (CRD) was taken and repeated 4 times.
Application dosage: 2.4 billion nematodes/ha., 1.2 billion nematodes/ha., 0.6 billion nematodes/ha.,

Each pot of grass was shaven 2 days before applying the worms. Twenty plantlets were examined in each pot. The bitetrace numbers on the top 6 leaves of each plantlet were calculated as the numbers of affected grasses and leaves.

Every pot of grass was irrigated with 400 ml running water before the application of the pesticide and worm, and was irrigated with 150 ml running water after the application to facilitate the release of nematode SCA from vermiculite. The results are listed in Table 3.

**Table 3**

| The effects of the biological controlling on tobacco cutworm by SCA cultured in vermiculite in the pot test | | | | | |
|---|---|---|---|---|---|
| Treatment dosage (per hect) | repeat | number of damaged leaves | X±DS% | number of damaged grass | X±DS% |
| 2.4 x 10⁹ | 1 | 1 | 4±2.4 b 3.3 | 1 | 2±1.5 c 10 |
| | 2 | 5 | | 2 | |
| | 3 | 2 | | 1 | |
| | 4 | 6 | | 4 | |
| 1.2 x 10⁹ | 1 | 13 | 14±3.4 b 11.7 | 12 | 8±2.6 b 40 |
| | 2 | 10 | | 6 | |
| | 3 | 13 | | 8 | |
| | 4 | 15 | | 7 | |
| 0.6 x 10⁹ | 1 | 43 | 37±11.2 a 30.8 | 13 | 14±3.7 a 70 |
| | 2 | 38 | | 14 | |
| | 3 | 21 | | 9 | |
| | 4 | 46 | | 15 | |
| 0 | 1 | 15 | 31±12.9 a 25.8 | 8 | 14±3.9 a 65 |
| | 2 | 44 | | 17 | |
| | 3 | 26 | | 14 | |
| | 4 | 38 | | 15 | |

Data was analyzed by One Way ANOVA and tested by Duncan's Multiple Range test. The different numbers behind means represent the significant difference between treatments (p= 0.05).

The numbers of damaged leaves and grasses were calculated by random selection of 20 plantlets and the top 6 leaves of each plantlet, so the total number of leaves to be calculated was 120 leaves/per pot.
(I) As shown in Table 1, a high yield of nematode can be obtained when using porous materials. The yield obtained by using sponge as culture material was the highest.
(II) As shown in Table 2, the addition of symbiotic bacteria facilitated mass rearing of nematode SCA. The yield of culture was 1.2 billion/vessel. The pesticidal activity at day 5 was 95%.
(III) The effects of the biological controlling on tobacco cutworm by SCA cultured in vermiculite in the pot test were as follows:

As shown in Table 3, the treatment at 2.4 x 10⁹/ha 1.2 x 10^{9/}ha were significantly better than that at 6 x 10⁸/ha and control. For the damage of grass, the controlling effects were 2.4 x 10⁹/ha > 1.2 x 10⁹/ha > 6 x 10⁸ha. Nematode SCA presented in vermiculite preparation demonstrated the effect on preventing tobacco cutworm to destroy lawn. Therefore, the method for culturing nematode of the present invention produced at a high yield, and the insecticides made by the method have high pesticidal activity and preventive effects.

Moreover, the storage for the biotic preparations could be more than 6 months under refrigerated condition.

Although the examples of present invention have been described and illustrated in the specification and figures, but it should be appreciated that all of them are only the exemplification of the concepts and characters of present invention, and not for limiting the scopes of present invention and claims.

## Claims

1. A biotic preparation for controlling insects which comprises (a) an environmentally compatible porous material, and (b) beneficial nematodes and symbiotic bacteria which have been incubated in a production medium therefor in the porous material.

2. A biotic preparation according to claim 1, wherein said environmentally compatible porous material is vermiculite, peat or pearlite.

3. A biotic preparation according to claim 1 or 2, wherein said environmentally compatible porous material is biodecomposable.

4. A biotic preparation according to any one of the preceding claims wherein said environmentally compatible porous material is in the form of granules.

5. A biotic preparation according to any one of the preceding claims wherein said symbiotic bacteria is *Xenorhabdus or Photorhabdus*.

6. A biotic preparation according to claim 5, wherein said *Xenorhabdus* is *Xenorhabdus nematophilus*.

7. A biotic preparation according to any one of the preceding claims wherein said nematodes are monoxenic or sterilised.

8. A biotic preparation according to any one of the preceding claims wherein said nematodes are *Steinernema carpocapsae*.

9. A method for preparing a biotic preparation which method comprises providing an environmentally compatible porous material as defined in any one of claims 1 to 4 containing entomogenous nematodes with pesticidal activity, symbiotic bacteria and production medium therefor.

10. A method according to claim 9, wherein said symbiotic bacteria is as defined in claim 5 or 6.

11. A method according to claim 9 or 10 wherein said nematodes are as defined in claim 7 or 8.

12. A method according to any one of claims 9 to 11 also comprising mixing the symbiotic bacteria and the nematodes and inoculating the porous material with the mixture.

13. A method for controlling pests at a locus comprising providing at the locus an environmentally compatible porous material as defined in any one of claims 1 to 4 containing beneficial nematodes with pesticidal activity.

14. A method according to claim 14 wherein the environmentally compatible porous material containing beneficial nematodes with pesticidal activity is a biotic preparation according to any one of claims 1 to 8 or a biotic preparation which has been prepared by a method according to any one of claims 9 to 12.

15. A method according to claim 13 or 14, wherein said locus is grass, a field, a pot or a lawn.
